# DEMANDE DE BREVET EUROPEEN

(11) **EP 4 477 756 A1**
(43) Date de publication de la demande: **18.12.2024**
(21) Numéro de dépôt: 24178897.5
(22) Date de dépôt: 29.05.2024
(51) Int. Cl.: C12Q 1/04, G01N 33/487

(54) **PROCÉDÉ DE DÉTECTION ET D'IDENTIFICATION DE MICRO-ORGANISMES DANS UN ÉCHANTILLON PAR FRAGMENTATION DE L'ÉCHANTILLON**

(30) Priorité: 15.06.2023 FR 2306139
(71) Demandeur: Commissariat à l'Energie Atomique et aux Energies Alternatives, 75015 Paris (FR)
(72) Inventeur: BABIN, Thibaut, 38054 Grenoble cedex 09 (FR); MARCOUX, Pierre, 38054 GRENOBLE Cedex 09 (FR); MAILLEY, Pascal, 38054 Grenoble cedex 09 (FR); GOUGIS, Maxime, 38054 GRENOBLE Cedex 9 (FR); MERMET, Xavier, 38054 Grenoble cedex 09 (FR)
(74) Mandataire: INNOV-GROUP

(57) **Abrégé**

L'invention concerne un Système de détection, adapté à la mise en oeuvre d'un procédé de détection de la présence de plusieurs micro-organismes et d'identification desdits micro-organismes dans un même échantillon, ledit procédé comportant des étapes de :
a. Fragmentation (E2) de l'échantillon (ECH) en plusieurs sous-échantillons,
b. Placement (E3) de chaque sous-échantillon (S1-S10) dans un compartiment (C1-C10) distinct, lesdits compartiments contenant un même milieu de culture et étant instrumentés chacun à l'aide d'une électrode de mesure (E10) et d'au moins une électrode de référence (E20),
c. Mesure (E4) au cours du temps dans chaque compartiment de la différence de potentiel entre l'électrode de mesure (E10) et l'électrode de référence (E20),
d. Détermination du profil de la dérivée de la différence de potentiel pour chaque sous-échantillon (S1-S10),
e. Détection (E5) de la présence ou non de chaque micro-organisme présent dans chaque sous-échantillon (S1-S10) et identifier la nature de chaque micro-organisme présent, à partir du profil obtenu à l'étape d),

## Description

### Domaine technique de l'invention

La présente invention se rapporte à un procédé de détection de la présence d'au moins un micro-organisme au sein d'un échantillon, par fragmentation de l'échantillon en plusieurs sous-échantillons. L'invention concerne également un système de détection apte à mettre en oeuvre le procédé.

### Etat de la technique

Le sang est en temps normal totalement stérile. Tout micro-organisme présent dans le système sanguin représente donc une menace vitale pour le corps humain. L'hémoculture est actuellement une méthode connue de diagnostic des infections sanguines. Il s'agit d'un test fréquent en microbiologie clinique. L'hémoculture consiste en premier lieu en la culture du sang dans un bouillon nutritif en condition aérobie et anaérobie, sous incubation à 36-37°C dans des automates adaptés à ce type d'échantillons, afin d'obtenir une croissance bactérienne. L'objectif consiste à amplifier la quantité de bactéries présentes dans l'échantillon, en lui fournissant des conditions favorables de croissance, et ainsi à détecter la présence de bactérie dans le sang chez un patient présentant un état septique. Cette amplification est d'autant plus nécessaire que la concentration bactérienne dans le sang au cours des bactériémies est toujours faible, de l'ordre d'une ou de quelques bactéries par millilitre de sang prélevé. Cette première étape de l'hémoculture consiste donc en une simple détection de présence, sans identification, à partir d'un échantillon sans flore normale : toute présence de bactérie ou champignon conduit donc à un test positif.

Cette détection sera ensuite suivie d'une culture sur milieu gélosé pour isoler le pathogène sous forme de colonies, permettant ensuite de procéder à l'identification du pathogène et à son antibiogramme. Dans le cas des infections sanguines, les examens sont urgents et le délai de rendu des résultats (positivité de l'hémoculture, identité du pathogène, antibiogramme) a un impact indéniable sur le devenir des patients (mortalité, durée d'hospitalisation, complications ...). Les résultats d'hémoculture permettent en effet d'adapter l'antibiothérapie au cas spécifique du patient : plus une antibiothérapie adaptée et efficace intervient précocement, plus les chances de survie du patient s'accroissent. Chaque heure de retard à l'instauration d'une antibiothérapie adaptée est associée à une augmentation de la mortalité.

Les industriels du diagnostic ont oeuvré pendant de nombreuses années à réduire le délai de positivité des hémocultures, ainsi qu'à réduire les temps d'analyse sur les tests d'identification et d'antibiogramme. Actuellement, deux grands types d'automates coexistent :
- Des appareils qui suivent la quantité d'acide carbonique généré dans la phase liquide, grâce à une matrice polymère (silicone) chargée avec un chromophore ou un fluorophore sensible au pH.
- Des appareils employés pour déceler une augmentation de la pression totale dans la phase gaz.

Ces deux technologies ont en commun d'être mises en oeuvre dans des automates, souvent de grande taille, avec l'impossibilité de démarrer le test tant que le flacon n'est pas dans l'automate (une pré-incubation peut générer des faux-négatifs), d'où la perte de précieuses heures avant la mise en place d'une antibiothérapie optimisée. Une étude en 2013 a montré que le temps de transport moyen était de 9 h (écart interquartile : 3-15 h), avec 6% de flacons présentant un temps de transport supérieur à 20 h.

La demande de brevet EP4018191A1 décrit l'utilisation d'un conteneur instrumenté doté d'électrodes de mesure, pouvant accueillir un échantillon liquide à analyser. La détection de présence de micro-organismes est notamment réalisable par électrochimie, en plaçant le conteneur dans un caisson adapté. Cette demande de brevet propose une solution simple permettant de réduire la durée entre le prélèvement de l'échantillon et le démarrage de l'analyse de l'échantillon prélevé. Cette solution est par ailleurs facilement transportable, la rendant disponible sur le terrain, et facile à mettre en oeuvre, même par du personnel peu qualifié.

Une méthode récente (demande de brevet FR2114688) permet de détecter et d'identifier la croissance d'un microorganisme dans un échantillon liquide grâce aux signatures potentiométriques enregistrées au cours du temps. Cette méthode est particulièrement adaptée lorsqu'une unique espèce bactérienne est présente dans l'échantillon et permet un temps de détection plus faible lorsque la concentration bactérienne est plus élevée. En revanche, dans le cas d'une contamination polymicrobienne (au moins deux espèces de microorganismes différentes dans le même échantillon), les signatures électrochimiques générées ne permettent d'identifier que l'espèce qui se multiplie le plus rapidement, la colonisation des électrodes interdisant ensuite l'apparition d'une nouvelle signature potentiométrique indiquant la présence d'une deuxième espèce.

Les publications référencées ci-dessous proposent des solutions de fragmentation d'échantillon :
- SZYLOWSKI L. et AL. « High-throughput 96-well bioelectrochmical platform for screening of electroactive microbial consortia", et
- KUKENBUCH Anne et Al. "Electrochmeical Microwell Plate to Study Electroactive Microorganisms in Parallel and Real-Time"

Le but de l'invention est de proposer une solution pour fragmenter rapidement un échantillon en vue de son analyse.

### Exposé de l'invention

Ce but est atteint par un système de détection, adapté à la mise en oeuvre d'un procédé de détection de la présence de plusieurs micro-organismes et d'identification desdits micro-organismes dans un même échantillon, ledit procédé comportant des étapes de :
a. Fragmentation de l'échantillon en plusieurs sous-échantillons,
b. Placement de chaque sous-échantillon dans un compartiment distinct, lesdits compartiments contenant un même milieu de culture et étant instrumentés chacun à l'aide d'une électrode de mesure et d'au moins une électrode de référence,
c. Mesure au cours du temps dans chaque compartiment de la différence de potentiel entre l'électrode de mesure et l'électrode de référence,
d. Détermination du profil de la dérivée de la différence de potentiel pour chaque sous-échantillon,
e. Détection de la présence ou non de chaque micro-organisme présent dans chaque sous-échantillon et identifier la nature de chaque micro-organisme présent, à partir du profil obtenu à l'étape d).

Ledit système comprenant un dispositif de mesure à plusieurs compartiments, chaque compartiment étant destiné à recevoir un sous-échantillon distinct, caractérisé en ce que chaque compartiment est instrumenté à l'aide d'au moins une électrode de mesure et d'une électrode de référence, le dispositif de mesure se présentant sous la forme d'un unique flacon dont le volume interne comporte des cloisons agencées pour former lesdits compartiments.

Selon une particularité, le flacon comporte une entrée commune et un répartiteur disposant de plusieurs canaux d'écoulement débouchant chacun dans un compartiment distinct du flacon.

Selon une autre particularité, chaque électrode de mesure est réalisée sous la forme d'un dépôt d'une encre conductrice ou d'un électro-dépôt d'un élément de mesure conducteur.

Selon une autre particularité, ladite électrode de référence est réalisée sous forme d'un dépôt d'une encre Ag/AgCI recouverte d'une couche polymère.

Selon une autre particularité, ledit échantillon est du sang.

Le micro-organisme est par exemple choisi dans le groupe constitué par les microorganismes procaryotes tels que les archées ou les bactéries Gram+ ou Gram-, les microorganismes eucaryotes comme les levures et autres champignons microscopiques et les protistes comme les algues ou protozoaires.

### Brève description des figures

D'autres caractéristiques et avantages vont apparaître dans la description détaillée qui suit faite en regard des dessins annexés dans lesquels :
- La figure 1 illustre le principe de réalisation du procédé de l'invention ;
- La figure 2 illustre le principe de fragmentation d'un échantillon ;
- La figure 3 illustre le principe de fragmentation d'un échantillon et permet de mettre en évidence la détection de deux bactéries A et B au sein des fragments de cet échantillon ;
- Les figures 4A et 4B montrent un premier exemple de réalisation du dispositif de mesure de l'invention, respectivement vu de côté et vu de dessus d'une coupe transversale ;
- Les figures 5A et 5B montrent un deuxième exemple de réalisation du dispositif de mesure de l'invention, respectivement vu en perspective et vu de dessus ;

### Description détaillée d'au moins un mode de réalisation

L'invention vise notamment un procédé facile à mettre en oeuvre, rapide et efficace, grâce auquel il est possible de détecter mais aussi d'identifier les micro-organismes contenus dans un échantillon liquide ou solide et ce, en utilisant l'électrochimie.

Ce procédé repose notamment sur le principe d'une fragmentation de l'échantillon en plusieurs sous-échantillons.

Pour la détection, l'invention s'appuie sur une variation de signal électrochimique et notamment sur l'évolution de la dérivée du potentiel électrochimique par rapport au temps. Avec un traitement du signal adapté, il est possible de détecter la croissance d'un micro-organisme comme une croissance bactérienne et d'orienter l'identification.

L'échantillon ECH peut être un fluide biologique, avantageusement choisi dans le groupe constitué par le sang tel que du sang entier ou du sang entier anti-coagulé, le sérum sanguin, le plasma sanguin, la lymphe, les larmes, le sperme, le liquide céphalo-rachidien, le liquide interstitiel, un liquide articulaire, un liquide péricardique, un fluide isolé de moelle osseuse, un extrait cellulaire, un extrait tissulaire, un extrait d'organes et un de leurs mélanges. Ainsi, le fluide biologique peut être tout fluide naturellement sécrété ou excrété d'un corps humain ou animal, ou tout fluide récupéré, à partir d'un corps humain ou animal, par toute technique connue de l'homme du métier telle qu'une extraction, un prélèvement, une ponction ou un lavage. Les étapes de récupération et d'isolement de ces différents fluides à partir du corps humain ou animal sont réalisées préalablement et ne font pas partie de l'invention.

L'échantillon ECH peut également être un produit liquide issu d'une industrie agro-alimentaire, pharmaceutique ou cosmétique. Dans un mode de réalisation particulier, l'objet sur lequel le prélèvement est effectué peut être choisi parmi des installations de grande taille comme un objet industriel, comme un appareil électronique ou une machine utilisée dans les industries agro-alimentaires, pharmaceutiques ou cosmétiques, une cuve, une cuisine de restaurant, une chambre froide, un sanitaire, un conteneur, et des objets de petite taille comme des dispositifs médicaux ou des tuyaux.

L'échantillon ECH peut également être un milieu de culture stérile, entourant un échantillon solide ou pulvérulent dont on veut tester la stérilité, par exemple une biopsie, des tissus pour greffes, un dispositif médical (valve cardiaque, prothèse, lentille de contact, seringue, aiguille, etc.), un principe actif en poudre, du matériel de laboratoire à usage unique, un équipement chirurgical...

Dans un mode de réalisation plus particulier, l'échantillon ECH est liquide et est constitué de sang, comme du sang humain ou animal. Ce dernier est normalement stérile mais peut contenir des micro-organismes comme des bactéries.

En effet, des bactéries peuvent être présentes dans le sang suite à des actes théoriquement inoffensifs tels que le brossage de dents, des soins dentaires ou des actes médicaux impliquant des sondes ou des cathéters. On parle alors de bactériémie non pathologique. La bactériémie pathologique correspond, quant à elle, à la présence de bactéries dans le sang suite à une pneumonie, une plaie ou une infection urinaire.

Parmi les micro-organismes contenus ou susceptibles d'être contenus dans l'échantillon, on trouve aussi bien des micro-organismes procaryotes tels que les archées ou les bactéries Gram+ ou Gram-, des micro-organismes eucaryotes comme les levures et autres champignons microscopiques et les protistes comme les algues ou protozoaires. A titre d'exemples illustratifs et non limitatifs de micro-organismes contenus ou susceptibles d'être contenus dans l'échantillon mis en oeuvre dans le cadre de l'invention, on peut citer les bactéries de la famille des *Pseudomonadaceae,* les bactéries du genre *Staphylococcus,* les bactéries du genre *Streptococcus,* les bactéries du genre *Campylobacter,* les bactéries du genre *Haemophilus,* les bactéries du genre *Anaerococcus,* les bactéries du genre *Bacteroides,* les bactéries du genre *Acinetobacter,* les bactéries du genre *Stenotrophomonas,* les bactéries du genre *Pasteurella,* les bactéries du genre *Bacillus,* les bactéries du genre *Listeria,* les bactéries du genre *Clostridium,* les bactéries du genre *Mycobacteria,* les bactéries du genre *Enterococcus* et les bactéries du genre *Pasteurella,* les levures du genre *Candida,* les levures du genre *Saccharomyces,* les champignons du genre *Aspergillus,* les champignons du genre *Pénicillium*
De plus, parmi les bactéries de l'ordre des *Enterobacterales* contenues ou susceptibles d'être contenues dans l'échantillon mis en oeuvre dans le cadre de l'invention, on peut citer les bactéries des genres *Escherichia, Salmonella, Shigella, Enterobacter, Klebsiella, Serratia, Proteus, Morganella, Yersinia, Citrobacter* et *Providencia.*

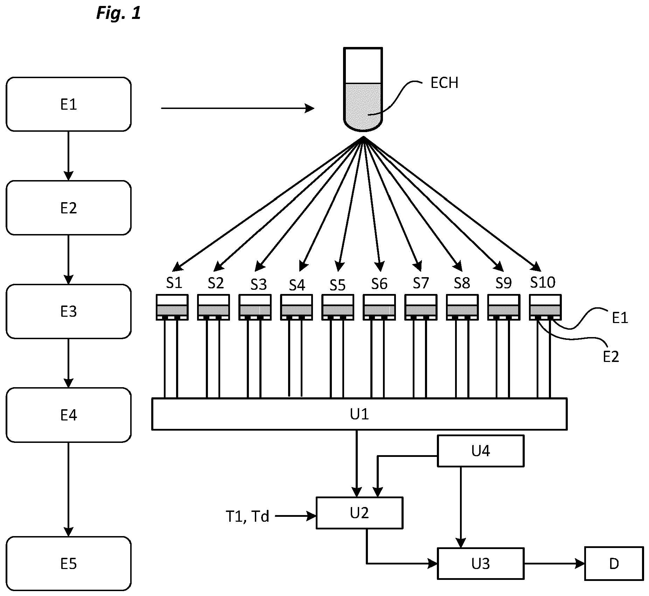

En référence à la figure 1, le procédé de l'invention comporte les étapes décrites ci-dessous.

### Première étape E1

La première étape E1 consiste à prélever un échantillon ECH à analyser. A titre d'exemple, on prélève 10mL de sang sur un patient.

### Deuxième étape E2

La deuxième étape E2 consiste à fragmenter l'échantillon ECH prélevé en plusieurs sous-échantillons S1, S2, S3, S4, S5, S6, S7, S8, S9, S10.

La fragmentation de l'échantillon ECH en plusieurs sous-échantillons S1-S10 permet de diminuer le temps de détection, en augmentant la concentration microbienne dans un fragment (par réduction du volume). Elle présente également l'intérêt de rendre l'échantillon ECH plus lisible dans le cas d'une infection polymicrobienne.

La fragmentation de l'échantillon permet également de diminuer le temps de détection en enregistrant des négatifs (les sous-échantillons sans bactérie) relatifs au patient. On dispose ainsi d'une ligne de base du signal personnalisée, permettant une détection plus fine/rapide.

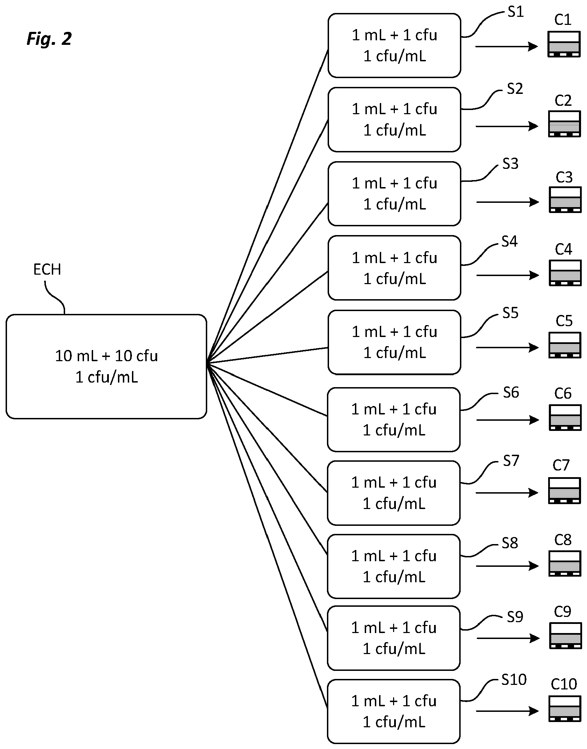

La figure 2 illustre le cas classique d'une hémoculture. Dans ce cas, la charge bactérienne du sang d'un patient atteint de bactériémie est en moyenne de 1 cfu/mL (cfu - "colony forming unit"). Pour une hémoculture, le prélèvement est de 10mL. Il contient donc potentiellement 10 cfu.

En fragmentant les 10 mL de sang ici en 10 volumes de 1 mL, on obtient une disparité des concentrations avec deux situations extrêmes :
- Les bactéries sont réparties parfaitement équitablement,
- Les bactéries sont toutes dans le même compartiment.

Dans ces deux cas, la diminution du volume permettra une réduction du temps de détection (la croissance microbienne suivant une loi exponentielle au moment de la détection).

Dans la majorité des cas (donc hors cas extrêmes), on retrouve des fragments plus concentrés et d'autres sans bactérie du tout (comme on peut le voir sur la Figure 2).

Si le patient est atteint d'une infection polymicrobienne (au moins deux espèces bactériennes), la fragmentation de l'échantillon ECH est également un avantage : avec suffisamment de divisions, il est possible de séparer les deux espèces et ainsi de ramener la détection au cas classique d'une infection mono-microbienne.

Le nombre de fragments de l'échantillon ECH est une donnée à étudier statistiquement, pour maximiser les chances de séparer deux espèces lors d'une infection polymicrobienne. D'un point de vue statistique, le nombre de fragments/divisions doit être choisi pour que la probabilité d'avoir 2 cfu dans le même sous-échantillon soit très faible (de l'ordre de 1%). Grâce à la loi de Poisson, nous savons que dix divisions sont nécessaires (au minimum) pour une concentration bactérienne de la prise d'essai de 1 cfu/mL.

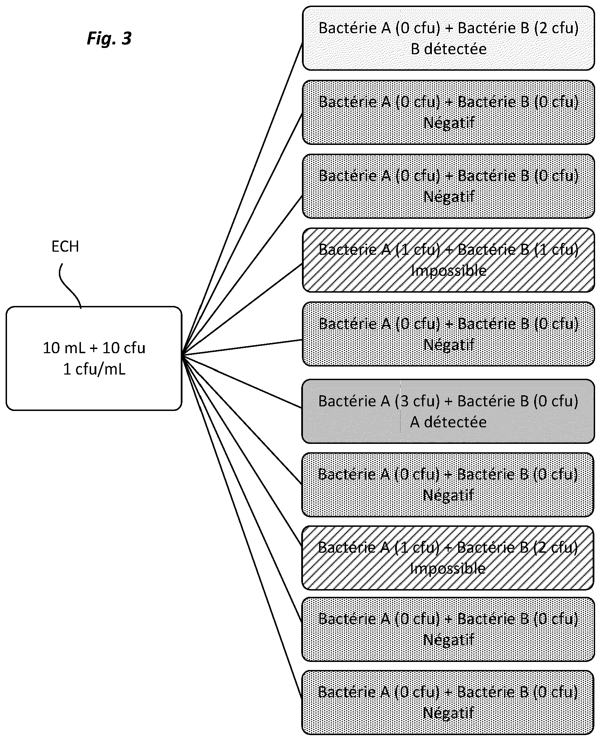

La figure 3 illustre ce principe dans le cas d'un échantillon prélevé contenant une première bactérie A (5 cfu) et une deuxième bactérie B (5 cfu). Cet échantillon ECH est fragmenté en dix sous-échantillons S1-S10. La figure 3 montre un exemple de répartition possibles des deux bactéries dans les dix sous-échantillons créés. On constate que :
- Un sous-échantillon S6 ne comporte que la bactérie A ;
- Un sous-échantillon S1 ne comporte que la bactérie B ;
- Certains sous-échantillons S2, S3, S5, S7, S9, S10 ne comportent aucune bactérie (négatif) ;
- Certains sous-échantillons S4, S8 comportent les deux bactéries A et B (aucune identification possible) ;

### Troisième étape E3

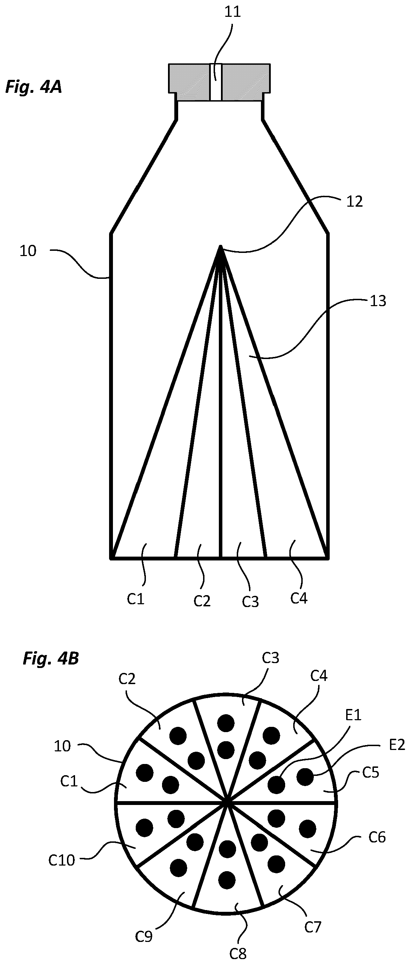

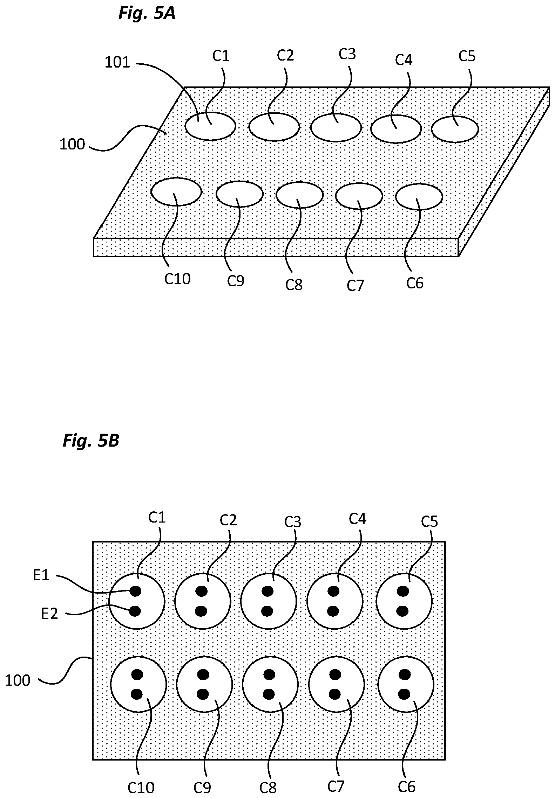

Chaque sous-échantillon S1-S10 est placé dans un compartiment C1-C10 distinct d'un dispositif de mesure, chaque compartiment contenant un milieu de culture liquide. Des exemples de réalisation d'un dispositif de mesure compartimenté sont donnés ci-après, en liaison avec la figure 4A, figure 4B, figure 5A et figure 5B.

Le milieu de culture liquide est identique pour tous les compartiments C1-C10 et donc pour tous les sous-échantillons S1-S10. Les compartiments sont isolés de manière étanche les uns des autres.

Chaque compartiment C1-C10 est instrumenté à l'aide de moyens de mesure électrochimique.

Les moyens de mesure comportent au moins deux électrodes E10, E20 différentes, utilisées pour analyser le sous-échantillon placé dans le compartiment, une électrode de mesure E10 et une électrode de référence E20.

Les électrodes E10, E20 sont par exemple intégrées au compartiment de sorte que le liquide qui y est contenu vienne au contact des électrodes, lors de l'analyse.

L'électrode de mesure E10 utilisée est avantageusement une électrode réalisée sous forme d'un dépôt d'une encre conductrice ou une électrode réalisée sous forme d'un électro-dépôt d'un élément de mesure conducteur. Toute encre conductrice connue de l'homme du métier peut être mise en oeuvre. Dans un mode de réalisation particulier, l'encre conductrice utilisée est une encre à base d'ions métalliques ou de polymères organiques conducteurs tels que le polythiophène (PT), la polyaniline (PANI) éventuellement dopée avec de l'acide dodecylbenzènesulfonique (DBSA), le poly(3,4-éthylènedioxythiophène) couplé au polystyrène sulfonate) de sodium (PEDOT:PSS), le polypyrrole ou le polyphthalocyanine. Dans un autre mode de réalisation particulier, l'encre conductrice utilisée dans le cadre de l'invention est une encre carbone comprenant éventuellement un élément de mesure conducteur additionnel. Par "élément de mesure conducteur", on entend un élément de mesure choisi dans le groupe constitué par les polymères organiques conducteurs tels que ceux listés ci-dessus, et les composés à base de métal comme, par exemple, un oxyde métallique tel que l'oxyde d'iridium (IrOx), un pigment à base de métal tel que le bleu de Prusse (Fe(lll) 5 ferrocyanure) ou encore un catalyseur organique ou organo-inorganique tel que de la phtalocyanine de cobalt.

Avantageusement, l'électrode de référence E20 utilisée dans le cadre de l'invention est réalisée sous forme d'un dépôt d'une encre Ag/AgCI.

Le dispositif de mesure compartimenté est intégré dans un système de détection configuré pour détecter et éventuellement identifier les micro-organismes contenus dans chaque sous-échantillon contenu dans chaque compartiment.

Le système comporte également :
- Une unité de mesure U1 de la différence de potentiel entre l'électrode de mesure E10 et l'électrode de référence E20 et configurée pour mesurer dans chaque compartiment la différence de potentiel, en continu et/ou à une pluralité d'instants temporels,
- Une unité électronique d'acquisition U2 de la mesure de la différence de potentiel, reliée à l'unité de mesure,
- Une unité de traitement U3 reliée à l'unité électronique d'acquisition U2 et configurée pour traiter la mesure de la différence de potentiel,
- Une unité d'alimentation électrique U4 pour alimenter l'unité électronique d'acquisition U2 et éventuellement l'unité de traitement U3.

Une unité de chauffage peut également être ajoutée, ainsi qu'une unité de mesure et de gestion de la température. Le chauffage devra être réalisé de manière homogène, tout autour du conteneur. L'unité de mesure et de gestion de la température peut comporter une sonde de température et des moyens de régulation de ladite température à une valeur fixe déterminée. La valeur de température est choisie en fonction du type de culture mise en oeuvre. Dans le cas d'une hémoculture, la température d'incubation est choisie égale à 35°C +/- 2°C.

L'unité d'alimentation électrique U4 peut être constituée d'une batterie rechargeable destinée à alimenter les différentes unités du dispositif.

L'unité de traitement U3 peut comporter un microprocesseur et des moyens de mémorisation. Elle peut également comporter un module de communication. Ce module peut être sans-fil pour communiquer via une liaison sans-fil, par exemple selon le protocole Bluetooth, avec le module correspondant de l'unité d'acquisition.

A titre d'exemple, l'unité de traitement U3 peut être configurée pour :
- Recevoir les données de mesure de la différence de potentiel en provenance de l'unité d'acquisition, par exemple via la liaison sans-fil,
- Etablir un suivi électrochimique du mélange en fonction du temps,
- Calculer la dérivée de la différence de potentiel acquise,
- Traiter la courbe obtenue pour déterminer la présence de micro-organismes,
- Commander une interface homme-machine destinée à indiquer la présence de bactéries,
- Lire une étiquette RFID présente sur le conteneur 1 en vue de récolter et de stocker les données liées au prélèvement,
- Assurer la régulation de l'unité de chauffage pour assurer une température d'incubation la plus constante possible.

La signature électrochimique obtenue pour chaque compartiment permettra de repérer s'il s'agit d'une division mono-microbienne (signature classique), d'une division négative (pas de détection) ou d'une division polymicrobienne (signature bruitée).

### Quatrième étape E4

Cette étape E4 du procédé correspond, pour chaque compartiment C1-C10, au suivi de l'évolution, dans le milieu de culture liquide, de la différence de potentiel entre l'électrode de mesure E10 et l'électrode de référence E20.

La mesure de la différence de potentiel se fait de façon continue ou discontinue. Dans le mode discontinue, la différence de potentiel est mesurée à une pluralité d'instants temporels distincts.

L'intervalle entre l'instant temporel T1 de la première mesure et l'instant temporel Td de la dernière mesure de potentiel est variable et fonction notamment de l'échantillon à étudier, des micro-organismes susceptibles d'être présents dans ce dernier et de l'instant temporel t1. Avantageusement, l'instant temporel td de la dernière mesure de potentiel est réalisé au moins 14 h, au moins 16 h, au moins 18 h, au moins 20 h ou encore au moins 22 h après le début de la troisième étape E3.

### Cinquième étape E5

Cette étape est mise en oeuvre par l'unité de traitement U3 et consiste à calculer la dérivée du potentiel électrochimique mesurée. L'unité de traitement U3 effectue ce calcul pour chaque compartiment et donc pour chaque sous-échantillon.

En effet, afin d'observer plus nettement le début des variations de potentiel lorsque des micro-organismes provenant de l'échantillon ECH se développent dans le milieu de culture, la dérivée de la différence de potentiel pour chaque électrode est utilisée. Ainsi, on observe la vitesse de modification du potentiel, beaucoup plus parlante et lisible que les courbes potentiométriques brutes.

La détection de la présence ou non de micro-organismes dans l'échantillon ECH peut se faire en comparant le profil de la dérivée du potentiel ou éventuellement le profil normalisé de la dérivée de la différence de potentiel avec le profil de la dérivée du potentiel ou éventuellement le profil normalisé de la dérivée de la différence de potentiel, obtenu avec un contrôle négatif c'est-à-dire dénué de micro-organismes et dans les mêmes conditions opératoires et notamment même(s) électrode(s) de travail et de référence et même milieu de culture.

L'identification d'un micro-organisme particulier peut se faire en comparant le profil de la dérivée de la différence de potentiel ou éventuellement le profil normalisé de la dérivée du potentiel avec différents profils de la dérivée du potentiel ou éventuellement différents profils normalisés de la dérivée de la différence de potentiel obtenu, chacun, avec un micro-organisme connu et dans les mêmes conditions opératoires et notamment même(s) électrode(s) de travail et de référence et même milieu de culture.

Pour chaque compartiment, on obtient alors un diagramme de résultats similaire à celui de la figure 3.

Il faut noter que dans chaque compartiment C1-C10, après avoir déterminé la positivité dans un compartiment (c'est-à-dire la présence de bactéries dans le sous-échantillon), l'identification est possible si le sous-échantillon ne comporte qu'une seule bactérie. Si le sous-échantillon comporte par exemple deux types de bactéries distincts, l'identification peut rester possible si l'une des bactéries croît plus vite que l'autre. Dans ce cas, il reste en effet possible d'identifier une signature électrochimique. Dans le cas où les deux bactéries croissent à la même vitesse, l'identification sera difficile.

### Dispositif de mesure

Comme indiqué ci-dessus, le système de détection comporte un dispositif de mesure adapté à la mise en oeuvre du procédé de l'invention.

Deux exemples de réalisation du dispositif de mesure sont présentés ci-dessous.

Dans un premier exemple de réalisation, le dispositif de mesure se présente sous la forme d'un flacon 10 dont le volume interne est compartimenté. Il comporte par exemple autant de compartiments C1 -C10 que de fragments de l'échantillon réalisé à la deuxième étape. Chaque compartiment C1-C10 est séparé d'un compartiment adjacent par une cloison.

Il faut noter que le dispositif pourrait comporter un plus grand nombre de compartiments qu'il y a de sous-échantillon, par exemple pour effectuer des mesures sur des échantillons de référence.

Chaque compartiment du flacon 10 est instrumenté de manière à intégrer au moins deux électrodes (électrode de mesure E10 + électrode de référence E20).

Le flacon 10 peut présenter des moyens pour permettre de diviser l'échantillon ECH en plusieurs fragments. Il faut noter que les différents fragments obtenus ne sont pas forcément de volumes identiques. Le flacon 10 comporte par exemple une entrée 11 commune menant à un répartiteur 12. Le répartiteur 12 dispose de plusieurs canaux 13 d'écoulement, débouchant chacun dans un compartiment distinct.

Le flacon 10 comporte par exemple sur sa face externe, des électrodes de reprise de contact, permettant de relier chaque électrode de mesure E10 et chaque électrode de référence E20 à l'unité de mesure. Sur ce point, il peut s'agir du principe déjà décrit dans la demande de brevet WO2021/032654A1**.**

Dans un deuxième exemple de réalisation, le dispositif de mesure comporte une plaque 100 à puits, chaque puits 101 formant un compartiment C1-C10 distinct destiné à recevoir un sous-échantillon S1-S10. Chaque puits 101 est instrumenté à l'aide d'au moins une électrode de mesure E10 et une électrode de référence E20.

Dans cette variante, le dispositif comporte un répartiteur fluidique contrôlé pour délivrer un fragment de l'échantillon ECH dans chaque puits 101 de la plaque 100.

De manière non limitative la plaque 100 à puits peut être constituée d'une première plaque dotée de trous traversants contre laquelle est appliquée une deuxième plaque portant les électrodes et venant former le fond de chaque puits.

De manière identique, les électrodes de mesure E10 et de référence E20 associés à chaque compartiment de la plaque à puits sont reliées à l'unité de mesure via des liaisons électriques.

Il faut noter que plus les mesures sont effectuées sur un nombre de fragments de l'échantillon important, plus la détection est sensible. Autrement dit, le dispositif de mesure doit comporter suffisamment de compartiments instrumentés, adaptés au volume d'échantillon prélevé.

L'invention présente ainsi de nombreux avantages, parmi lesquels :
- Une détection fiable et sensible, même en cas d'infection polymicrobienne ;
- Une détection plus rapide, permise par la fragmentation de l'échantillon, celle-ci augmentant la concentration microbienne dans un fragment et permettant également l'obtention de négatifs (sous-échantillons sans bactérie) ;
- Un système de détection doté d'un dispositif de mesure adapté à la mise en oeuvre du procédé ;
- L'utilisation d'un dispositif de mesure approprié tel que le flacon compartimenté ;

## Revendications

1. Système de détection, adapté à la mise en oeuvre d'un procédé de détection de la présence de plusieurs micro-organismes et d'identification desdits micro-organismes dans un même échantillon, ledit procédé comportant des étapes de :
a. Fragmentation (E2) de l'échantillon (ECH) en plusieurs sous-échantillons,
b. Placement (E3) de chaque sous-échantillon (S1-S10) dans un compartiment (C1-C10) distinct, lesdits compartiments contenant un même milieu de culture et étant instrumentés chacun à l'aide d'une électrode de mesure (E10) et d'au moins une électrode de référence (E20),
c. Mesure (E4) au cours du temps dans chaque compartiment de la différence de potentiel entre l'électrode de mesure (E10) et l'électrode de référence (E20),
d. Détermination du profil de la dérivée de la différence de potentiel pour chaque sous-échantillon (S1-S10),
e. Détection (E5) de la présence ou non de chaque micro-organisme présent dans chaque sous-échantillon (S1-S10) et identifier la nature de chaque micro-organisme présent, à partir du profil obtenu à l'étape d),
Ledit système comprenant un dispositif de mesure à plusieurs compartiments (C1-C10), chaque compartiment (C1-C10) étant destiné à recevoir un sous-échantillon (S1-S10) distinct, **caractérisé en ce que** chaque compartiment (C1-C10) est instrumenté à l'aide d'au moins une électrode de mesure (E10) et d'une électrode de référence (E20), le dispositif de mesure se présentant sous la forme d'un unique flacon (10) dont le volume interne comporte des cloisons agencées pour former lesdits compartiments (C1-C10).

2. Système selon la revendication 1, **caractérisé en ce que** le flacon (10) comporte une entrée (11) commune et un répartiteur (12) disposant de plusieurs canaux (13) d'écoulement débouchant chacun dans un compartiment distinct du flacon.

3. Système selon la revendication 1 ou 2, **caractérisé en ce que** chaque électrode de mesure (E10) est réalisée sous la forme d'un dépôt d'une encre conductrice ou d'un électro-dépôt d'un élément de mesure conducteur.

4. Système selon l'une des revendications 1 à 3, **caractérisé en ce que** ladite électrode de référence (E20) est réalisée sous forme d'un dépôt d'une encre Ag/AgCI recouverte d'une couche polymère.

5. Système selon l'une des revendications 1 à 4, **caractérisé en ce que** ledit échantillon est du sang.
